# EUROPEAN PATENT APPLICATION

(11) **EP 4 686 478 A1**
(43) Date of publication of application: **04.02.2026**
(21) Application number: 24382848.0
(22) Date of filing: 31.07.2024
(51) Int. Cl.: A61K 38/08, A61P 3/10, C07K 7/06

(54) **QBP1 FOR USE IN THE TREATMENT AND/OR PREVENTION OF TYPE 2 DIABETES**

(71) Applicant: Consejo Superior de Investigaciones Científicas (CSIC), 28006 Madrid (ES)
(72) Inventor: Carrión Vázquez, Mariano Sixto, 28002 Madrid (ES); Tejero Ojeda, María del Mar, 28002 Madrid (ES)
(74) Representative: Pons IP

(57) **Abstract**

The present invention relates to the inhibitory effect of QBP1 on hIAPP amyloid formation disrupting the initial stages of hIAPP fibrillogenesis and mitigating hIAPP-induced islet amyloidosis toxicity. The invention relates to the peptide QBP1, or composition comprising thereof, for use in the treatment and/or prevention of the diabetes, particularly type 2 diabetes.

## Description

The invention relates to the peptide QBP1, or composition comprising thereof, for use in the treatment and/or prevention of the diabetes, particularly type 2 diabetes. The invention can be circumscribed to the Medicine field.

### BACKGROUND ART

Amyloid diseases are a heterogeneous group of disorders in which proteins self-assemble into misfolded soluble and insoluble molecular structures, such as oligomers and fibrils, which often exert toxicity on cells. The aggregation-prone human islet amyloid polypeptide (hereinafter, hlAPP or amylin) is a major component of amyloid deposition found in pancreatic islets of type 2 Diabetes (T2D) patients (Reed J., et al., 2021, A review of current trends with type 2 diabetes epidemiology, aetiology, pathogenesis, treatments and future perspectives. Diabetes, Metab. Syndr. Obes. 14, 3567-3602). This 37-amino acid residue peptide, co-secreted with insulin by pancreatic β-cells, plays a crucial role in regulating glucose homeostasis by inhibiting insulin and glucagon secretion and modulating satiety (Ling W, et al., 2019, Human Amylin: From Pathology to Physiology and Pharmacology. Curr. Protein Pept. Sci. 20, 944-957).

Although the hlAPP monomer is not inherently cytotoxic in humans, the oligomers and fibrils formed during its amyloidogenic process contribute to β-cell failure, depletion, and ultimately cell death, thereby accelerating the onset and progression of T2D (Suárez R., et al., 2023, Epigenetics in Obesity and Diabetes Mellitus: New Insights. Nutrients 15, 1-15). Oligomers, in particular, are identified as the most toxic intermediates, contributing to membrane disruption, endoplasmic reticulum stress, and mitochondrial damage (Kiriyama and Nochi, 2018, Role and cytotoxicity of amylin and protection of pancreatic islet β-cells from amylin cytotoxicity. Cells 7, 14-16)*.* The hypersecretion of hlAPP is associated with atypical Ca²⁺ ions release and activation of proteolytic enzymes, which contributes to the progressive destruction of β-cells in T2D (Alrouji et al., 2023, The potential role of human islet amyloid polypeptide in type 2 diabetes mellitus and Alzheimer's diseases. Diabetol. Metab. Syndr. 15, 1-16).

Despite the clear correlation between the degree of islet amyloid deposition and T2D severity, conflicting views still persist regarding the specific hIAPP's role in the etiology of this multifactorial disease (Alrouji et al., 2023, The potential role of human islet amyloid polypeptide in type 2 diabetes mellitus and Alzheimer's diseases. Diabetol. Metab. Syndr. 15, 1-16). Regarding the aggregation mechanism, it has been shown that the hydrophobic features of hIAPP (associated to particular amino acid residues such as isoleucine, alanine, leucine, and phenylalanine), promote the clustering of these residues and facilitate its aggregation (Fortier M., et al., 2022, Contribution of the 12 - 17 hydrophobic region of islet amyloid polypeptide in self-assembly and cytotoxicity 1-20). Additionally, "amyloidogenic hotspots", which encourage the formation of β-sheet-rich folding intermediates, have been identified within amylin's sequence (Mietlicki-Baase, E.G., 2018, Amylin in Alzheimer's disease: Pathological peptide or potential treatment? Neuropharmacology 176, 139-148). Thus, critical β-sheet domains, including the 8-20 or 30-37 sequences capable of self-aggregation along with the 20-29 sequence, have been identified as potential contributors to fibril formation (Unnikrishnan and Shanmugam, 2022, Isotope-edited vibrational circular dichroism study reveals a flexible N-terminal structure of islet amyloid peptide (NFGAIL) in amyloid fibril form: A site-specific local structural analysis. J. Struct. Biol. 214, 107910). In contrast, rodent amylin, which differs from its human counterpart by six residues, lacks the propensity to form amyloid fibrils (Kiriyama and Nochi, 2018, Role and cytotoxicity of amylin and protection of pancreatic islet β-cells from amylin cytotoxicity. Cells 7, 14-16). This finding highlights the significance of residues 22-29 (NFGAILSS) for human IAPP fibril formation. Notably, proline substitutions at positions 25, 28, and 29 destabilize hIAPP's β-sheet structure, which constitutes the conceptual basis of the FDA-approved drug Pramlintide, the pioneering therapeutic amylin analogue authorized for both type 1 and type 2 diabetes (T1D and T2D) (Boyle, C.N., et al., 2022, Mediators of Amylin Action in Metabolic Control. J. Clin. Med. 11).

Over the last few years, there has been an unprecedented progress in the development of protein aggregation inhibitors (Fernández Ramirez et al., 2023, Conformational inhibitors of protein aggregation. Curr. Opin. Struct. Biol. 83, 102700). Thus, numerous short-peptide inhibitors have been engineered to target specific intermediate species in the fibrillogenesis process or amyloidogenic hotspots within amyloid proteins (Murakami H., et al., 2023, Recent Advances in Drug Therapy for Parkinson's Disease. Intern. Med)*.* Overall, these therapies are highly coveted for their promising outcomes, characterized by high specificity, low toxicity, rapid blood clearance, swift cell and tissue permeability, and excellent *in vivo* biocompatibility with low immunogenicity (Wang C., et al., 2023, Advances in Alzheimer's Disease-Associated Aβ Therapy Based on Peptide. Int. J. Mol. Sci. 24).

Examples of such peptide-based inhibitors with demonstrated ability to inhibit hlAPP aggregation *in vitro* include SNNFGA, GAILSS, NYGAILSS, and NFGAILPP, which were developed against specific human amylin regions (Kapurniotu A., et al., 2002, Structure-based design and study of non-amyloidogenic, double N-methylated IAPP amyloid core sequences as inhibitors of IAPP amyloid formation and cytotoxicity. J. Mol. Biol. 315, 339-350). Another potent inhibitor, D-ANFLVH, effectively prevents islet amyloid aggregation and deposition, leading to reduced β-cell apoptosis, preserved β-cell region, and improved glucose tolerance in a T2D mouse model (Wijesekara N., et al., 2015, Islet amyloid inhibitors improve glucose homeostasis in a transgenic mouse model of type 2 diabetes. Diabetes, Obes. Metab. 17, 1003-1006).

On the other hand, numerous peptide-based inhibitors featuring diverse structural elements, including proline, α-Aminoisobutyric acid (Aib) and α, β-dehydrophenylalanine (AF), have been extensively documented in the literature. An interesting example is the aforementioned Pramlintide, a medication that has garnered significant success, overcoming hIAPP's tendency to aggregate and reducing surface adhesion (Boyle C.N., et al., 2022, Mediators of Amylin Action in Metabolic Control. J. Clin. Med. 11). This amylin analogue complements insulin effects by regulating postprandial glucose levels through decreased glucagon secretion (McQueen and Bonk, 2005, Pramlintide acetate. Am. J. Heal. Pharm. 62, 2363-2372). However, this drug faces significant drawbacks such as the cost of preparation, solubility issues, and limited oral bioavailability (Alrefai et al., 2010, Pramlintide: Clinical strategies for success. Diabetes Spectr. 23, 124-130). Furthermore, other recently developed amylinomimetics show promise with improved efficacy and prolonged duration of action. All these findings highlight the potential of short-peptide inhibitors in mitigating the pathophysiological effects associated to hIAPP fibrillogenesis.

The polyglutamine-binding peptide 1 (QBP1) was identified through a phage display screening targeting large polyQ stretches associated with polyQ diseases, such as Huntington's disease (Nagai Y., et al., 2000, Inhibition of polyglutamine protein aggregation and cell death by novel peptides identified by phage display screening. J. Biol. Chem 275, 10437-10442). It has been shown that QBP1 inhibits the toxic conformational transition of expanded polyQ tracts and subsequent oligomer formation (Popiel H.A., et al., 2011, The Aggregation Inhibitor Peptide QBP1 as a Therapeutic Molecule for the Polyglutamine Neurodegenerative Diseases. J Amino Acids. 265084)*.* Its anti-amyloidogenic activity has been demonstrated *in vitro* and, in both *Drosophila* (Nagai Y., et al., 2003, Prevention of polyglutamine oligomerization and neurodegeneration by the peptide inhibitor QBP1 in Drosophila. Hum Mol Genet 12, 1253-1259), and mouse models (Yang M., et al., 2018, Brain-Targeting Delivery of Two Peptidylic Inhibitors for Their Combination Therapy in Transgenic Polyglutamine Disease Mice via Intranasal Administration. Mol Pharm 15, 5781-5792; Popiel, H.A., et al., 2007, Protein transduction domain-mediated delivery of QBP1 suppresses polyglutamine-induced neurodegeneration in vivo. Mol. Ther. 15, 303-9). However, its exogenous administration to a mouse model of polyQ disease was unsuccessful, likely due to proteolysis and inefficient delivery across the blood-brain barrier (BBB) (Popiel H.A., et al., 2013, Inhibition of Protein Misfolding/Aggregation Using Polyglutamine Binding Peptide QBP1 as a Therapy for the Polyglutamine Diseases. Neurotherapeutics 10, 440-446)*.* Beyond polyQ diseases, QBP1 has shown anti-amyloidogenic inhibitory effects in other pathological amyloids, including α-synuclein (α-Syn) (Hervás R., et al., 2012, Common features at the start of the neurodegeneration cascade. PLoS Biol. 10, 1-32) and TAR DNA-binding protein 43 (TDP-43) as well as some functional amyloids such as Sup35 (Mompeán et al., 2019, Molecular mechanism of the inhibition of TDP-43 amyloidogenesis by QBP1. Arch. Biochem. Biophys 675, 108113) and the neuronal CPEB family (Ramírez de Mingo et al., 2023, Phase separation modulates the functional amyloid assembly of human CPE83. Prog. Neurobiol. 231). Still, it must be noted that the amyloid aggregation of other amyloids, such as amyloid-β 42 (Aβ42) and tau protein, was not affected by QBP1 (Hervás R., et al., 2012, Common features at the start of the neurodegeneration cascade. PLoS Biol. 10, 1-32*;* Fernández-Ramírez MdC., et al., 2023, Expanded Conformations of Monomeric Tau Initiate Its Amyloidogenesis. Angew. Chem. Int. Ed. Engl. 62(19): e202209252). Recent NMR studies suggest that QBP1's aromatic rings could disrupt intramolecular hydrogen bonding of asparagine and glutamine residues in TDP-43 amyloid nuclei, which supports its therapeutic potential for Amyotrophic Lateral Sclerosis (ALS) and Frontotemporal Dementia (FTD) (Mompeán et al., 2019, Molecular mechanism of the inhibition of TDP-43 amyloidogenesis by QBP1. Arch. Biochem. Biophys 675, 108113).

However, it is still necessary to find alternative compounds to protect pancreatic β-cells from the cytotoxic effects induced by hlAPP amyloidogenesis and inhibit the formation of islet amyloids and halt the onset and progression of T2D.

### DESCRIPTION OF THE INVENTION

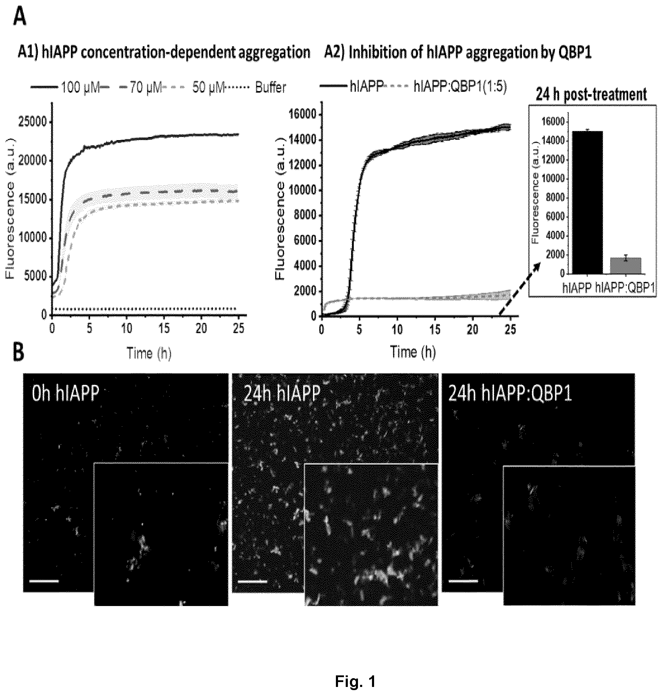

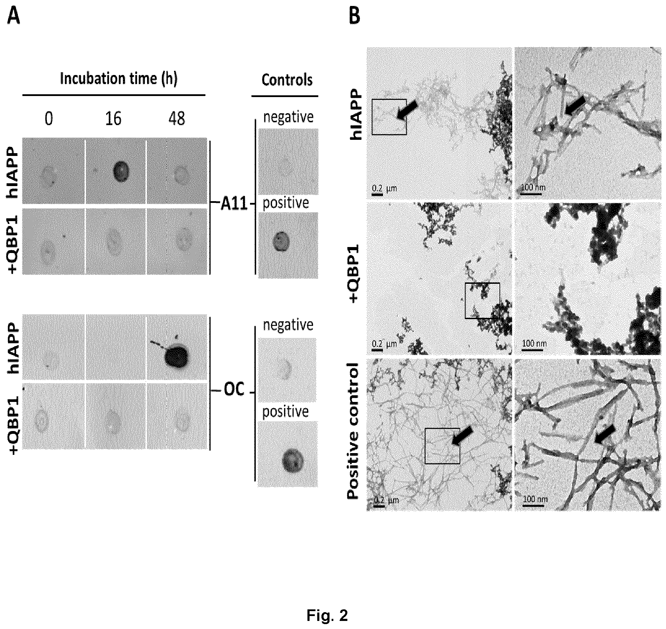

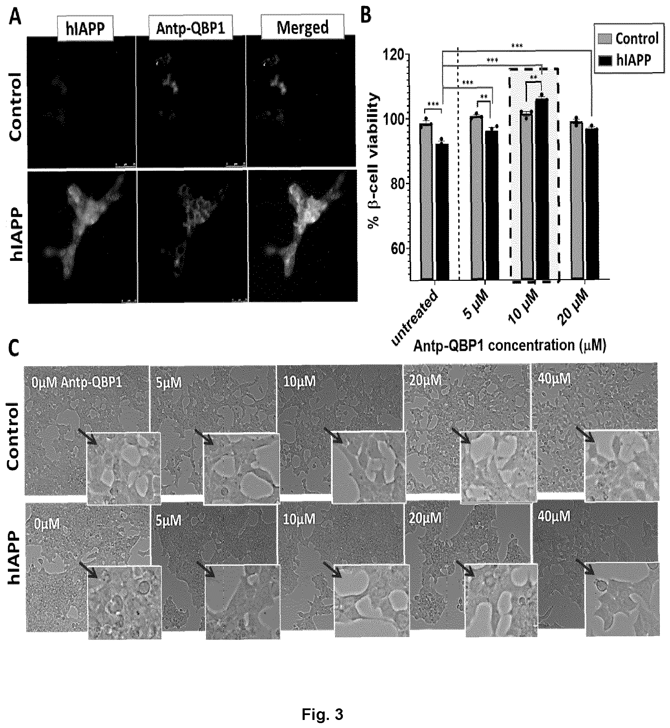

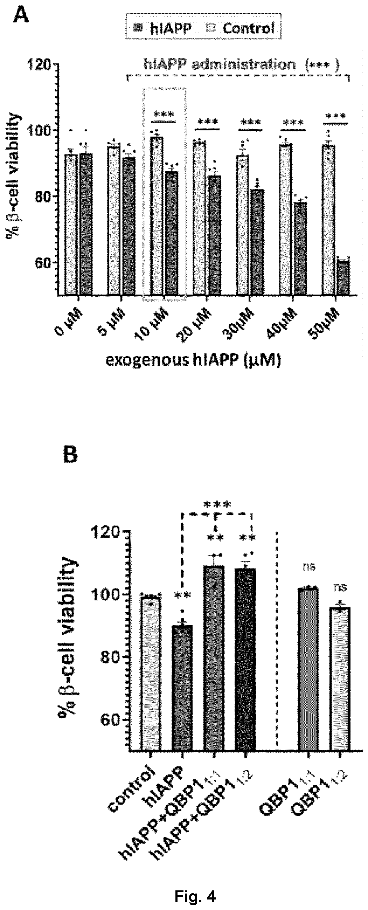

The present invention relates to the inhibitory effect of QBP1 on hlAPP amyloid formation disrupting the initial stages of hlAPP fibrillogenesis and mitigating hIAPP-induced islet amyloidosis toxicity. The inventors have conducted time-resolved kinetic studies using Thioflavin T (ThT) (Figure 1). Additionally, immunodot-blot analysis using conformational antibodies and transmission electron microscopy were employed to observe alterations in the morphology and abundance of hlAPP oligomers and fibrils species following treatment with QBP1 (Figure 2). Furthermore, the inventors have tested whether QBP1 is able to protect INS-1 pancreatic β-cells from the cytotoxic effects induced by hlAPP (Figure 3). To this end, for the delivery of QBP1 into the affected β-cells, the inventors have fused it with a protein transduction domain (PTD), specifically the *Drosophila* Antennapedia (Antp) PTD (Popiel H.A., et al., 2009, Delivery of the aggregate inhibitor peptide QBP1 into the mouse brain using PTDs and its therapeutic effect on polyglutamine disease mice. Neuroscience Letters 449, 87-92), to efficient cell membrane penetration in a receptor-independent manner (Figure 4). The findings demonstrate that QBP1 significantly reduces the formation of oligomeric and fibrillar species produced during hlAPP fibrillogenesis and mitigates the cytotoxic impact of hlAPP in INS-1 pancreatic β-cells. These results highlight the potential of QBP1, particularly its minimal active sequence (Tomita K., et al., 2009, Structure-activity relationship study on polyglutamine binding peptide. BMCL 17, 1259-1263), with amino acid sequence WKWWPGIF (SEQ ID NO:1), as a promising therapeutic intervention to inhibit the formation of islet amyloids and halt the onset and progression of diabetes, particularly type 2 diabetes mellitus (T2DM) also known as type 2 diabetes (T2D).

In support of the non-predictability of the results backing the invention, the following considerations must be taken into account: 1) the specific mechanism of action of the polyvalent amyloidogenic inhibitor QBP1 is still unknown; 2) QBP1 it works with several amyloids (both Q/N-rich and not Q/N-rich) but not with all of them, but currently there is no way to predict whether it will work with a certain amyloid or not since there is no predictor (neither sequence-based or conformation-based); 3) the specific target recognized by QBP1 (the pharmacophore) is also unknown, although a plausible working hypothesis is that since there is no sequence motif shared by all the amyloids it inhibits, it is expected that it is a conformational (not a sequence) inhibitor; 4) the molar ratio that works for amylin (1:5; amylin:QBP1) is lower than the one that works for CPEB3 (1.8) for which we have shown that it works in animals (*Drosophila* and mouse), therefore, it is expected that it would also work in mice to inhibit amylin (even better than for CPEB3) and is therefore a good candidate for therapeutic intervention (prevention and treatment) in Type 2 Diabetes.

Finally, based on the capability of the peptide to block the amyloidogenesis of amylin and considering both the high sequence homology with β-amyloid (52% similarity, 37% identity, according to a BLAST search) and their propensity for co-aggregation or cross-seeding, the present invention refers also to a method with potential to prevent neurodegenerative diseases associated to DT2, like Alzheimer's disease, by targeting amylin.

A first aspect of the invention refers to a peptide comprising an amino acid sequence with at least a 70% of identity with SEQ ID NO: 1 (wherein the sequence SEQ ID NO: 1 is the QBP1 peptide with sequence WKWWPGIF, hereinafter "the peptide of the invention"), for use in the treatment and/or prevention of diabetes, hereinafter "the use of the invention".

Therefore, the present invention also refers to the use of the peptide comprising an amino acid sequence with at least a 70% of identity with SEQ ID NO: 1, for the manufacture of a medicament for the treatment and/or prevention of diabetes.

The term "identity", as used herein, refers to the percentage of identical amino acid residues between two peptides when they are compared. The sequence comparison methods are known in the art, and include but are not limited to, the BLASTP or BLASTN, and FASTA program ClustalW. It can be considered that peptides identity percentages of at least 70% maintained the same properties of said polypeptide.

The percentage of identity of the peptide of the invention is of at least 70%; preferably the percentage of identity is 71, 72, 73, 74, 75, 76, 77, 78, 79, 80, 81, 82, 83, 84, 85, 86, 87, 88, 89, 90, 91, 92, 93, 94, 95, 96, 97, 98, 99 or 100%.

Preferably the peptide of the invention comprises the amino acid sequence SEQ ID NO: 1, more preferably consists of the sequence SEQ ID NO: 1.

The results obtained can be extrapolated to the complete QBP1, the 11-residue peptide (SEQ ID NO: 2, SN**WKWWPGIF**D), or derivatives of. Thus, in another preferred embodiment of the use of the invention, the peptide comprises an amino acid sequence with at least 70% of identity with SEQ ID NO: 2; more preferably, comprises the sequence SEQ ID NO: 2; and in a preferred embodiment the amino acid sequence consists of SEQ ID NO: 2.

In general, the skilled in the art will appreciate that deletions or substitutions may be made to the amino acid sequences of peptides of the present invention without unduly adversely affecting the activity thereof. Thus, peptides containing such deletions or substitutions are a further aspect of the present invention. In peptides containing substitutions or replacements of amino acids, one or more amino acids of a peptide sequence may be replaced by one or more other amino acids wherein such replacement does not affect the function of that sequence. Such changes can be guided by known similarities between amino acids in physical features such as charge density, hydrophobicity/hydrophilicity, size and configuration, so that amino acids are substituted with other amino acids having essentially the same functional properties.

Therefore, as the skilled person in the art knows, the nucleotide sequence that codifies for the peptide of the invention could be useful in the treatment and/or prevention of diabetes. Herein the terms "nucleotide sequence", "polynucleotide", "nucleotide sequence", "nucleic acid" and "oligonucleotide" are used interchangeably. The term polynucleotide includes genomic DNA or DNA encoding double or single stranded, ribonucleic acid (RNA), any synthetic and genetically manipulated polynucleotide, and both sense and antisense strands. This includes single-chain molecules and double-stranded, such as DNA-DNA hybrids, DNA-RNA and RNA-RNA.

In a preferred embodiment of the use of the invention, the peptide is comprised in a composition, hereinafter "the composition of the invention". In the present invention the terms "composition", "pharmaceutical composition", "drug" and "medicament" are used interchangeably.

The term "pharmaceutical composition" herein refers to any substance used for prevention, diagnosis, alleviation, treatment or cure of diseases in humans or animals. The pharmaceutical composition of the invention can be used either alone or in combination with other pharmaceutical compositions. The term pharmaceutical composition and drug are used in this invention interchangeably. In the context of the present invention relates to a pharmaceutical composition or medicament characterized by comprising the peptide of the invention or the polynucleotide that codifies it allowing their expression in the organism to be treated in a therapeutically effective amount, such that the peptide of the invention performs its function in the target tissue or cell.

In a preferred embodiment of the invention, the composition further comprises an excipient and/or an acceptable pharmaceutical carrier and/or an adjuvant, or any combinations thereof.

The term "excipient" refers to a substance that helps absorption of the elements of the composition of the invention and actively stabilizes or assists the preparation of the composition in the sense of giving consistency or flavour. Thus, carriers may have the function of holding the ingredients together, such as in the case of starches, sugars or celluloses, sweetening function, the function as a colorant, the protective function of the composition, such as to isolate the air and/or moisture, filling the role of a tablet, capsule or other form of presentation, such as is the case of dibasic calcium phosphate, the disintegrating function to facilitate dissolution of the components and its absorption in the intestine, without excluding other excipients not mentioned in this paragraph.

The term "pharmaceutic carrier", refers to a substance used in the pharmaceutical composition or medicament to dilute any component of the present invention included therein to a given volume or weight. The vehicle function is to facilitate the incorporation of other elements, allow better dosage and administration or give substance and form to the composition. When the presentation is liquid, the pharmacologically acceptable carrier is the diluent.

Herein, the term "adjuvant" refers to an agent that increases the effect of the peptide of the invention when given jointly to it or forming part of the same treatment protocol. The pharmaceutically acceptable adjuvants and vehicles that may be used in the pharmaceutical composition of the present invention are vehicles known by those skilled in the art.

In a more preferred embodiment of the invention, the peptide additionally comprises an internalization agent.

The term "internalization agent" refers to a molecule capable of facilitating the passage of a given peptide through the intestinal barrier, blood-brain barrier (BBB) and the plasma membrane to permit access to the interior of cells. This molecule can be covalently bound or not to the peptide of the present invention. Non-limiting examples of the internalization agents include: Protein Transduction Domains (PTD) like Antp (penetratin, SEQ ID NO: 3, RQIKIWFQNRRMKWKK), TAT peptide (transactivator of transcription, SEQ ID NO: 4, YGRKKRRQRRR), VP22 peptide (from herpes simplex virus type 1) and Signal sequence-based peptides (SS peptides).

The composition provided by this invention can be administered by any suitable administration route, for which said composition is formulated in the suitable to the chosen route of administration pharmaceutical form.

In a more preferred embodiment of the invention, the composition is presented in an adapted form for oral, parenteral, intramuscular, intraarterial, intravenous, subcutaneous, epidural, intradermic intraperitoneal or intravesicular administration.

The peptide or the composition of the invention are used for the treatment and/or prevention of diabetes, preferably type 2 diabetes mellitus.

The term "treatment", as understood in the present invention, refers to combating the effects caused by a disease, in particular diabetes, in a subject (preferably a human), inhibiting the disease or pathological condition, in other words, stopping its development; alleviating the disease or pathological condition, in other words, causing the remittance of the disease or pathological condition or the symptoms thereof; or stabilising the disease or pathological condition.

The term "prevention" as understood in the present invention consists of preventing the onset of a disease, specifically diabetes, in other words, preventing the disease or pathological condition from occurring in a subject (preferably a human), in particular, when said subject has a predisposition to suffer from the disease.

The term "diabetes" refers to a group of metabolic disorders, whose main common characteristic is the presence of persistently or chronically high concentrations of glucose in the blood, due either to a defect in insulin production, to a resistance to its action in utilizing glucose, to an increase in glucose production, or to a combination of these causes. Diabetes disease include, without limitation, type 1 diabetes mellitus, type 2 diabetes mellitus or gestational diabetes.

In a preferred embodiment of the present invention, the diabetes is type 2 diabetes mellitus.

The present invention also refers to a method for the treatment and/or prevention of diabetes, preferably type 2 diabetes mellitus, that comprises the administration of a therapeutical dose of peptide of the invention, or composition comprises thereof, wherein the peptide comprises an amino acid sequence with at least a 70% of identity with SEQ D NO: 1 or SEQ D NO: 2, preferably comprises or consists of the sequence SEQ ID NO: 1 or SEQ ID NO: 2.

### DESCRIPTION OF THE DRAWINGS

**Figure 1****. Inhibition of early-stage hlAPP amyloid formation by QBP1 octapeptide.** (A) kinetics of hlAPP fibrillogenesis, monitored using the amyloid-sensitive fluorescent probe, Thioflavin T (ThT), over 24 hours. (A1) Increasing hlAPP concentrations (50 µM, 70 µM, and 100 µM) show a concentration-dependent acceleration in both the lag phase and growth phase of fibril formation compared to the buffer control. (A2) ThT fluorescence of hlAPP (70 µM) with and without QBP1 inhibitor (1:5 molar ratio). The presence of QBP1 significantly reduces ThT fluorescence, indicating inhibition of amyloid fibril formation from the onset. The bar chart on the right panel quantifies fluorescence emission levels after 24 h of treatment, showing a substantial decrease in the hlAPP + QBP1 sample compared to hlAPP alone. Data represents the average of three consecutive analyses. (B) Representative fluorescence microscopy images of ThT-stained samples. The hlAPP sample shows increasing fluorescence (in gray scale), indicative of amyloid fibril formation after 24 h. In contrast, hlAPP co-incubated with QBP1 (1:5 molar ratio) shows a marked reduction in fluorescence, suggesting effective inhibition of fibril formation. Insets highlight the fluorescence intensity at higher magnification. No fluorescence is detected in the initial 0-hour hlAPP sample. Scale bars: 75 µM).
**Figure 2****. QBP1 prevents the formation of toxic early-phase intermediates of hlAPP aggregation.** (A) Immunodot-blot analysis of hIAPP (70 µM) alone or co-incubated with QBP1 (1:5 molar ratio) at different incubation times (0, 16, and 48 hours) using A11 (oligomer-specific) and OC (fibril-specific) antibodies. Controls include bovine serum albumin (BSA) (non-amyloidogenic) as a negative control and amyloid-β 42 (amyloidogenic) as a positive control. hIAPP shows increasing reactivity with both A11 and OC over time, indicating progression from oligomeric to fibrillar species. QBP1 alone exhibits no reactivity, while hlAPP co-incubated with QBP1 significantly reduced A11 and OC reactivity, evincing inhibition of oligomer and fibril formation. (B) Representative TEM micrographs of hIAPP incubated alone, hlAPP co-incubated with QBP1 (1:5 molar ratio), and a positive control sample (α-synuclein fibrils). hIAPP forms dense fibrillar networks (indicated by black arrows), while QBP1-treated samples show a marked reduction in fibril formation after 72 h. α-Synuclein fibrils confirm the standard amyloid fibril structure by TEM. Insets provide higher magnification views to highlight the structural details of the aggregates. Scale bars: 0.2 µm (left) and 100 nm (right). The final data represent the accumulation of three consecutive analyses.
**Figure 3****. Antp-QBP1 effectively delivers QBP1 intracellularly and mitigates the cytotoxic effects induced by the overexpressed hIAPP in INS-1 β-cells.** (A) Immunofluorescence analysis of overexpressed hIAPP (first column) and Antp-QBP1 (second column) in hIAPP INS-1 and control (Ct) INS-1 β-cells. Cells were incubated with or without 10 µM Antp-QBP1 for 48 hours. The merged images (third column) illustrate the intracellular colocalization of hlAPP and Antp-QBP1 peptides. Images were acquired using a LEICA DMI 6000 Fluorescence Microscope with a 63x objective lens. (B) Cell viability analysis after 48 h of treatment with varying concentrations of Antp-QBP1 (5 µM, 10 µM, and 20 µM) on hlAPP INS-1 and Ct INS-1 β-cells. Antp-QBP1 significantly improves the viability of hIAPP-overexpressing cells at all tested concentrations compared to untreated cells (***p < 0.001), showing its protective effect against the cytotoxicity of hlAPP in pancreatic β-cells. Notably, the decrease in cell viability induced by hlAPP is reversed at 10 µM (**p < 0.002) and prevented at 20 µM by Antp-QBP1, enhancing viability in hIAPP-overexpressing cells relative to the treated Ct INS-1 β-cells. Cell viability was assessed using a luminescent assay (by a thermostable luciferase), with the luminescent signal corresponding to the number of viable cells. Results are presented as the mean ± SEM, with 3 replicates. (C) Representative light microscopy images of untreated and treated hlAPP INS-1 and Ct INS-1 β-cells with various concentrations of Antp-QBP1 (5 µM, 10 µM, 20 µM, and 40 µM) for 48 h. Untreated Ct INS-1 cells (top row) and hIAPP-overexpressing cells (bottom row) exhibit distinct cell morphologies, with the latter showing dense cytoplasm, loss of surface adhesion, rounding, and detachment. However, cells treated with Antp-QBP1 demonstrate restored adhesion and typical morphology. Insets show zoomed views to highlight detailed cell morphology. Images were obtained using a Leitz DM IRB microscope with a 10x/0.40 objective and a K5 camera (Leica, Germany). The scale bar represents 75 µm.
**Figure 4****. Antp-QBP1 enhances the cell viability reduced by exogenous administration of hIAPP prefibrillar oligomers in pancreatic INS-1 β-cells.** (A) Cell viability analysis of INS-1 β-cells treated with increasing hlAPP concentrations (ranging from 5 µM to 50 µM) for 48 h. The results show a dose-dependent reduction in cell viability caused by hlAPP prefibrillar oligomers compared to the buffer control (***p < 0.001), highlighting the cytotoxic effect of hlAPP on INS-1 β-cells. Notably, a significant decrease in cell viability is observed at 10 µM hIAPP (***p < 0.001), which was therefore selected for further testing. (B) Cell viability analysis of INS-1 cells treated with hlAPP alone (10 µM) or coincubated with two different concentrations of Antp-QBP1 (1:1 and 1:2, hIAPP: Antp-QBP1) for 48 h. All treatments showed a significant effect on cell viability compared to the buffer control (**P < 0.01). Specifically, Antp-QBP1 significantly enhances the cell viability reduced by exogenous administration of hlAPP in INS-1 β-cells (***P < 0.001) at both tested concentrations, demonstrating the protective effect of QBP1 against hlAPP-induced cytotoxicity. Cell viability was measured using a luminescent assay (by a thermostable luciferase), with the luminescent signal corresponding to the number of viable cells. Results are presented as the mean ± SEM, with 3 replicates.

### EXAMPLES

### Materials and Methods

### Preparation of the peptides

Synthetic human IAPP (1-37), as well as the QBP1 (octapeptide core, WKWWPGIF) and Antp-QBP1 peptides (Popiel et al., 2007, Protein transduction domain-mediated delivery of QBP1 suppresses polyglutamine-induced neurodegeneration in vivo. Mol. Ther. 15, 303-309), were synthesized by GenScript (Netherlands) ensuring a purity exceeding 95%. Following a disaggregation protocol (Arnés et al., 2020, PI3K activation prevents Aβ42-induced synapse loss and favors insoluble amyloid deposit formation. Mol. Biol. Cell 31, 244-260), synthetic hlAPP was dissolved in formic acid (FA) at the concentration of 0.5 mg/ml at room temperature (RT). After 3 minutes of sonication and subsequent vacuum drying, the dry FA-treated peptide was obtained. Stock solutions of QBP1 and Antp-QBP1 peptides were prepared in DMSO to a final concentration of 10 mM and stored at -80°C until further use.

### Amyloid formation

Oligomers and fibrils were prepared by dissolving the 0.5 mg hlAPP in 10 mM phosphate-buffered saline (PBS) at pH 7.4 containing 2% DMSO at the concentration of 100 µM, which was taken as the start of fibrillogenesis (i.e., designated as time-zero). After vortexing, hlAPP sample were incubated for 3 days at 25°C without shaking to promote the progressive oligomers and fibrils formation. Inhibitors, when used, were added at varying molar ratios relative to amylin at the beginning of the incubation to ensure their presence from the start of fibril fibrillogenesis. Aliquots were collected at different time points during incubation for simultaneous biochemical characterization and biological evaluation of QBP1 effects on INS-1 β-cells. Before using hlAPP oligomers for cytotoxicity assays, samples were sonicated to break and prevent incipient fibrils.

### Thioflavin T (ThT) Fluorescence Assay

Amyloid formation was monitored *in vitro* using time-resolved ThT-binding kinetics curves (Abedini et al., 2016, A Single-Point Mutation Converts the Highly Amyloidogenic Human Islet Amyloid Polypeptide into a Potent Fibrillization Inhibitor. J. AM. CHEM. SOC. 129, 11300-11301). Reactions were carried out in PBS pH 7.4 with 2% DMSO by mixing different concentrations of hIAPP peptide samples with 50 µM ThT (prepared from a 1 mM stock solution in PBS). The QBP1 inhibitor was used at five different molar ratios relative to amylin. Negative controls without QBP1 contained the same concentration of DMSO. Each experiment was repeated three times in 96-well plates. Fluorescence intensity (AU, arbitrary units) measurements were recorded over a 24-hour time course using a multifunctional spectrophotometer at 30°C, with excitation at 450 nm and emission at 482 nm. Additionally, fluorescence images of the same ThT microplate assays were captured using a LEICA DMI 6000 fluorescence microscope with a 10x objective. At least three images were analysed per experimental condition by using the Image J/Fiji software (NIH, USA).

### Dot blot immunoassay

Two µL of incubated samples at various time points were spotted onto a nitrocellulose membrane. After blocking non-specific interactions for 1 h at RT with 10% non-fat milk (Blotting-Grade Blocker, Bio-Rad) in Tris-buffered saline (TBS) containing 0.01% Tween 20 (TBS-T), the membrane was washed three times for 5 min with TBS-T and incubated for 1 h at RT with the A11 polyclonal anti-oligomer antibody (1:2000, ThermoScientific) (Kayed et al., 2003, Common structure of soluble amyloid oligomers implies common mechanism of pathogenesis. Science (80-.). 300, 486-489) and the fibril-specific OC polyclonal antibody (1:2500, Millipore) (Kayed et al., 2007, Fibril specific, conformation dependent antibodies recognize a generic epitope common to amyloid fibrils and fibrillar oligomers that is absent in prefibrillar oligomers. Mol. Neurodegener. 2, 1-11) in 5% non-fat milk in TBS-T. After three additional 5-minute washes with TBS-T, the membrane was incubated for 1 h with the IRDye 680LT anti-rabbit secondary antibody (LI-COR) and then revealed using an imaging system that employs near-infrared fluorescence detection technology. The final concentration of hlAPP was 70 µM). Positive and negative controls consisted of 66 µM Aβ42 fibrils and 60 µM BSA, respectively.

### Transmission Electron Microscopy

TEM was used to directly visualize the relative density and morphological changes of hlAPP amyloid fibrils pre- and post-treatment with QBP1. Briefly, 10 µL of incubated samples were adsorbed onto carbon-coated 300-mesh copper grids (Ted Pella Inc.) for 5 min, followed by negative staining with a 5% (w/v) aqueous uranyl acetate for another 5 min. After washing the grids twice with water and then air dried. Images were captured at both 25 and 100 kV of excitation voltages using a TEM Jeol 1011 transmission electron microscope equipped with a CCD Megaview III camera. Amyloid fibrils images from hlAPP were taken at a concentration of 70 µM after 72 h of incubation. Inhibitor, when present, was added at five different molar ratios relative to amylin.

### Cell Viability Assays

Rat INS-1 pancreatic β-cells (INS-1, hlAPP INS-1, and Ct INS-1 β-cells) were kindly provided by Anna Novials (IDIBAPS, Spain) (Hernández et al., 2018, Pancreatic β cells overexpressing hlAPP impaired mitophagy and unbalanced mitochondrial dynamics. Cell Death Dis. 9, 1-11). The hlAPP INS-1 β*-*cells were stably transfected to overexpress the human IAPP gene and compared with cells transfected with an empty vector (Ct INS-1 β-cells), used as a negative control.

To assess the anti-amyloidogenic effect of QBP1 in β-cells, the inventors employed the QBP1 core (sequence: WKWWPGIF, SEQ ID NO: 1) fused to Antennapedia (Antp) PTD (with sequence RQIKIWFQNRRMKWKK, SEQ ID NO: 3) (Popiel H.A., et al., 2007, Protein transduction domain-mediated delivery of QBP1 suppresses polyglutamine-induced neurodegeneration in vivo. Mol. Ther. 15, 303-309), capable of penetrating cell membranes efficiently in a receptor-independent manner. Additionally, this peptide was biotinylated at the N-terminus for subsequent detection and had two central glycine residues added as flexible linkers to prevent steric hindrance and maintain structural integrity. The small size and lack of a side chain of glycines would provide the necessary flexibility for proper peptide function and target interaction. Thus, the sequence used is the following: Biotin-SEQ ID NO: 5 (RQIKIWFQNRRMKWKKGGWKWWPGIF, SEQ ID NO: 5).

INS-1 β-cells were maintained in RPMI 1640 medium (Thermo Fisher), supplemented with 10% heat-inactivated fetal bovine serum (FBS, Gibco), 11 mM glucose, 10 mM Hepes, 2 mM L-glutamine, 1 mM sodium pyruvate, 50 µM β-mercaptoethanol, and 1% penicillin-streptomycin (Thermo Fisher), and incubated at 37°C with 5% CO₂. hlAPP INS-1 and Ct INS-1 β-cells were cultured under the same conditions, with the addition of 200 mg/ml Geneticin. For viability assays, cells were seeded at a density of 30,000 cells per well in 96-well plates 16 h before the start of the experiments. In order to determine the background luminescence, wells containing medium without cells was used. After 48 h of treatment with varying concentrations of Antp-QBP1 (5 µM, 10 µM, and 20 µM), cell viability was assessed using a luminescent Cell Viability Assay (CellTiter-Glo^{®}, Promega). This fast and sensitive method is based on producing cell lysis and generating, by a thermostable luciferase, a luminescent signal directly proportional to the amount of ATP released and, hence, to the number of viable cells. The plate was equilibrated at RT for 30 min, followed by mixing the CellTiter-Glo^{®} Buffer with the CellTiter-Glo^{®} Substrate. Subsequently, 100 µl of the resulting CellTiter-Glo^{®} Reagent were added in each well using a multichannel pipette. After 2 min of incubation at RT with shaking to induce cell lysis and 10 min of rest to stabilize the signal, luminescence was analysed by a multifunctional spectrophotometer. Additionally, INS-1 β-cells were imaged using bright-field microscopy pre- and post-treatment with Antp-QBP1 to assess changes in morphology. Images were acquired with 10x/0.40 objective using a Leitz DM IRB microscope (Leica, Germany) equipped with a Peltier temperature controller (Linkam, UK) and a K5 camera (Leica, Germany). The images were analyzed using Leica LAS AF Lite software.

For cytotoxicity assays involving exogenous hlAPP administration, INS-1 β-cells were cultured for 48 h in fresh medium containing lag-phase intermediates of h-IAPP or PBS-buffered negative control solutions, with or without Antp-QBP1. Samples were collected at specific time points during protein incubation and transferred into cultured INS-1 β-cells. Prior to transfer, hlAPP oligomers were sonicated to break and prevent incipient fibrils. The final peptide concentrations ranged from 10 to 50 µM after dilution in cell-based assays. Untreated negative control cells received an equivalent volume of fresh medium, and cell viability was assessed using a luminescent assay (by a thermostable luciferase), where the luminescent signal correlated with the number of viable cells.

### Immunocytochemistry

Immunocytochemistry (ICC) was employed to verify the correct expression of hlAPP in the hlAPP INS-1 β-cells and confirm its absence in the negative control (Ct INS1E), as well as to validate the internalization of the PTD-QBP1 peptide into cells. Briefly, INS-1 β-cells were cultured in 24-well plates onto 12 mm coverslips pre-treated with 1 mg/ml poly-D-lysine (PDL) in borate buffer pH 8.0 at a density of 200,000 cell/ml. For 48 h, the cells were incubated in culture medium containing biotin-tagged Antp-QBP1 (+Antp-QBP1, 10 µM) or buffered control solution. Subsequently, cells were fixed with 4 % paraformaldehyde (PFA) for 15 min at RT. Then, cells were permeabilized with 0.1 % Triton X-100 in PBS for 15 min and incubated with blocking solution (2 % BSA, 5 % normal goat serum (NGS), 0.1 % Triton-X100 in PBS) for 1 h. Subsequently, cells were incubated with primary antibodies (rabbit anti-human IAPP 1:400 from SIGMA, and mouse anti-Biotin 1:200 from VECTOR) overnight at 4°C, followed by incubation with fluorophore-conjugated secondary antibodies for 1 h at RT. The coverslips were mounted on slides with mounting medium. Immunofluorescence images to assess the immunopositivity of overexpressed hlAPP and Antp-QBP1 peptide were acquired using a Fluorescence Microscope LEICA DMI 6000 with a 63x objective. Image analysis was performed using Image J/Fiji software (NIH, USA), with at least three images analyzed per experimental condition.

### Statistical Analysis

The data values were expressed as mean ± SEM (standard error of the mean). Multiple comparisons were made by one-way or two-way analysis of variance (ANOVA) followed by Bonferroni post-hoc test. Differences were considered significant at p<0.05. Statistical analyses were performed using GraphPad Prism version 8.00 (GraphPad Software, La Jolla California, USA).

### Results

### QBP1 behaves as a potent inhibitor of hIAPP fibrillogenesis as revealed by time-resolved kinetics studies

To investigate the amyloidogenic behaviour of hlAPP and evaluate the inhibitory effect of QBP1 on its amyloid formation, the inventors conducted time-resolved ThT-binding kinetics studies (Figure 1). Amylin displays a characteristic macroscopic aggregation curve for amyloid fibril formation over time, comprising sequential phases including a lag phase, sigmoidal growth phase, and a final plateau phase. The heightened ThT fluorescent signal observed in the hlAPP sample, in contrast to the buffer control, confirms the formation of amyloid-like fibrils characterized by the β-sheet conformation within 24 hours of ThT analysis. In addition, there is a concentration-dependent acceleration observed in both the lag phase and the growth phase of the fibrillogenesis curves as the concentration of hlAPP are increased (50 µM, 70 µM, and 100 µM) (Fig. 1, A1). This indicates that higher concentrations of hlAPP promote quicker nucleation and elongation of amyloid fibrils. Remarkably, the addition of QBP1 inhibitor at a 5-fold molar excess relative to amylin before initiating the fibrillogenesis significantly decreased ThT fluorescence emission, indicating effective inhibition of amyloid-like fibril formation from the beginning of the process (Fig. 1, A2).

Furthermore, the inventors confirmed this result by visualizing each ThT-stained sample under fluorescence microscopy. Following 24 hours of incubation, the hlAPP sample displayed a gradual increase in flocculence and distinct strip-like fluorescence, characteristic of amyloid fibril formation. However, a remarkable decrease in fluorescence signal was observed when hlAPP was co-incubated with QBP1 (at a 1:5 molar ratio) (Fig. 1, B), indicating that QBP1 effectively inhibits its fibril formation. In contrast, the negative control group, which did not contain amyloidogenic hIAPP, showed no fluorescence signal at any time point, confirming the specificity of the ThT signal for amyloid structures.

These findings support QBP1's potential as an inhibitor of hlAPP amyloidogenesis *in vitro,* apparently by interfering with the nucleation and/or elongation phases of fibrillogenesis. Given this inhibition during early amyloidogenesis, QBP1 may block the formation of toxic early-phase intermediates such as oligomeric and prefibrillar species.

### In the presence of QBP1 no A11 and OC immunoreactivities were observed through the hIAPP aggregation process

To investigate QBP1's potential in halting the formation of toxic early-phase intermediates, the inventors analyzed the amyloid species formed during hlAPP fibrillogenesis using two standard conformational-specific antibodies: the A11 antibody, which recognizes prefibrillar oligomers, and the OC antibody, which targets fibrillar oligomers and mature amyloids. Additionally, transmission electron microscopy (TEM) was employed to directly visualize the relative density and morphological changes of hlAPP amyloid fibrils before and after treatment with QBP1 (Figure 2). The inventors found that hlAPP incubated alone exhibited increasing reactivity with both A11 and OC antibodies over time, indicating a progression from oligomeric to fibrillar species. Specifically, A11 immunoreactivity was evident after 16 hours, revealing the presence of prefibrillar oligomers, followed by OC positivity at 48 hours, indicating the emergence of mature fibrils.

Notably, co-incubation of hlAPP with QBP1 at a 5-fold molar excess resulted in a marked reduction in A11 and OC immunoreactivities, underscoring its inhibitory impact on oligomer and mature fibril formation. The negative control, bovine serum albumin (BSA), exhibited no immunoreactivity, confirming the specificity of A11 and OC signals for amyloid structures (Fig. 2A).

Furthermore, TEM analysis revealed the presence of mature amyloid fibrils formed by hlAPP after 72 hours of incubation. Conversely, samples co-incubated with QBP1 at a 5-fold molar excess relative to amylin exhibited a significant reduction in fibril abundance, predominantly displaying non-specific protein aggregation (Fig. 2, B). These findings further confirm the inhibitory effect of QBP1 on the formation of early-phase toxic intermediates, including oligomeric and prefibrillar species. All these data suggest that QBP1 inhibitor may act at the monomer level, preventing the misfolding of monomers and their subsequent aggregation into toxic oligomeric nuclei.

After having biochemically demonstrated the anti-amyloidogenic effect of QBP1 on hlAPP aggregation, the inventors proceeded to test *in vitro* the effects of this inhibitor using a cell-based system.

### QBP1 recues hIAPP cytotoxicity

The effect of QBP1 in a relevant cellular environment was tested in rat INS-1 β-cells overexpressing human IAPP (hIAPP INS-1) (Figure 3). Immunostaining validated the overexpression of hlAPP and demonstrated the efficient intracellular delivery of Antp-QBP1 into hlAPP INS-1 cells (Figure 3). Specifically, the immunolabelling of hlAPP was clearly observed in the cytoplasm, with staining intensity markedly stronger in hlAPP INS-1 cells compared to Ct INS-1 cells. For the analysis of peptide internalization, 10 µM Antp-QBP1 was added to the culture medium and incubated for 48 h, followed by extensive washing to remove peptides non-specifically adhered to the cell membrane. The inventors found that Antp-QBP1 was efficiently transduced into all cells and was localized more prominently in the cytoplasm than in the nucleus, as indicated by the high staining intensity observed. Thus, Antp-QBP1 readily enters the cells and remains stable in the cells and/or medium for at least 48 h. Remarkably, both peptides exhibited cytoplasmic colocalization in hlAPP INS-1 cells (Fig. 3A), suggesting a potential binding interaction between them.

Subsequently, based on the potential deleterious effects of transient hlAPP overexpression on pancreatic β-cells, the inventors investigated whether QBP1 could mitigate the associated cellular damage. For this purpose, the inventors employed a luminescent assay (by a thermostable luciferase) to measure cell viability (in this assay the luminescent signal correlates with the number of viable cells) on hlAPP INS-1 and Ct INS-1 β-cells after treatment with varying concentrations of Antp-QBP1, ranging from 5 µM to 20 µM). The inventors found significantly decreased viability in untreated hIAPP-expressing cells compared to control cells (***p < 0.001), aligning with previous findings that reported overexpression of hlAPP involving COS-1 cells also induced apoptosis *(*Haataja L., et al., 2008, Islet amyloid in type 2 diabetes, and the toxic oligomer hypothesis. Endocr. Rev. 29, 303-316*).* Therefore, the inventors suggest that the decreased viability could be closely associated to hlAPP overexpression, and therefore its amyloidogenesis, and could potentially be prevented by the QBP1 inhibitor.

Consequently, after 48 h of treatment with varying concentrations of Antp-QBP1 (5 µM, 10 µM, and 20 µM), the inventors observed a significant improvement in the viability of hIAPP-overexpressing cells at all tested concentrations compared to untreated cells (***p < 0.001), indicating a protective effect against hlAPP-induced cytotoxicity in pancreatic β-cells. Notably, the decrease in cell viability induced by hlAPP was reversed at 10 µM (**p < 0.002) and completely prevented at 20 µM by Antp-QBP1, resulting in enhanced viability of hIAPP-overexpressing cells relative to treated control INS-1 β-cells (Fig. 3, B).

Additionally, bright-field microscopy was employed to assess potential changes in cell morphology before and after Antp-QBP1 treatment. Untreated Ct INS-1 cells and hIAPP-overexpressing cells exhibited distinct cell morphologies, with the hIAPP-overexpressing cells showing dense cytoplasm, loss of surface adhesion, rounding, and detachment. However, cells treated with Antp-QBP1 displayed restored adhesion and typical morphology (Fig. 3, C). This outcome indicates that there is an improvement in cell viability when treated with Antp-QBP1.

In the experiments, the potential decrease in amyloidogenic aggregation of hlAPP by QBP1 parallels the decline in toxicity, but this does not prove that decreased toxicity is directly mediated by decreased aggregation. QBP1 may exert its effects by inhibiting interactions between hlAPP peptides and other molecules, and the decrease in aggregation may be an epiphenomenon. However, the findings are fully consistent with the ThT data and show the potential of QBP1 to mitigate hlAPP cytotoxic effects in this *in vitro* model of diabetes, i.e. the hlAPP INS-1 β-cells.

The inventors noted a notable enhancement in hlAPP INS-1 cell viability following treatment with 10 µM Antp-QBP1 (approximately 10%). To further validate that cytotoxicity stems from hlAPP amyloidogenesis and that cell rescue is attributed to the effect of Antp-QBP1, the inventors decided to enhance the cell model by exogenously administering hlAPP lag-phase intermediates to the INS-1 β-cell fresh medium and assessed the efficacy of Antp-QBP1 under these experimental conditions (Figure 4).

First, these experiments involved culturing INS-1 β-cell for 48h in fresh medium containing hlAPP lag-phase intermediates or PBS-buffered control solutions, with or without Antp-QBP1. To that end, samples collected from the hlAPP incubation reaction that showed A11 immunoreactivity (i.e., indicating the presence of hlAPP prefibrillar oligomers), were sonicated and transferred to cultured INS-1 β-cells. It was observed a significant dose-dependent decrease in INS-1 β-cell viability with the administration of increasing concentrations of hlAPP prefibrillar oligomers, ranging from 10 to 50 µM, compared to the buffer control (***p < 0.001), highlighting the cytotoxic effect of hlAPP on INS-1 β-cells. However, when 5 µM hlAPP was administered to fresh medium, no significant effect on INS-1 β-cell viability was observed compared to the control, which suggest that, under the experimental conditions, this is a suboptimal concentration. Notably, a significant decrease in INS-1 β-cell viability was already observed at 10 µM hlAPP (***p < 0.001), which was therefore selected for further testing (Fig. 4, A). These findings are consistent with previous reports suggesting that high doses of synthetic hlAPP (i.e., 10 µM) inhibited glucose-stimulated insulin secretion from isolated rat pancreatic islets, reduced pancreatic cell viability, and induced apoptosis *(*Kanatsuka et al., 2018, IAPP / amylin and β - cell failure : implication of the risk factors of type 2 diabetes. Diabetol. Int. 9, 143-157). Based on these findings, the inventors decided to maintain the concentration of 10 µM hlAPP for subsequent experiments involving the inhibitor rescue of cultured β-cells.

Finally, the inventors assessed the cytotoxic effects of exogenously administering 10 µM hlAPP prefibrillar oligomers to INS-1 β-cells, both alone and coincubated with either an equimolar concentration or a 2-fold molar excess of Antp-QBP1. Both peptides were co-incubated and simultaneously administered to fresh medium to ensure the inhibitor's effect during the early stages of hlAPP fibrillogenesis. After 48 hours of treatment, all treatments (10 µM hlAPP administrated alone and coincubated with Antp-QBP1) significantly affected INS-1 β-cell viability compared to the buffer control (**P < 0.01). Notably, it was observed a significant improvement in cell viability with both ratios of Antp-QBP1 (1:1 and 1:2, h!APP: Antp-QBP1), resulting in an increase of approximately 19% compared to the group treated with hlAPP alone (***P < 0.001) (Fig. 4, B) demonstrating the protective effect of QBP1 against hIAPP-induced cytotoxicity.

The results reinforce the notion that QBP1 is a potent polyvalent conformational inhibitor of amyloidogenesis, demonstrating comparable or superior efficiency for human IAPP compared to other target amyloids and other inhibitors. By effectively impeding amyloidogenesis right from its outset, QBP1 not only limits the formation of toxic species but also alleviates the deleterious impact of human IAPP amyloidogenesis on pancreatic cells. Consequently, QBP1 emerges as a promising therapeutic candidate for the prevention and management of diabetes, particularly type 2 diabetes, offering new hope in the search for effective treatments targeting this and other amyloid-related pathologies.

Notably, the low concentration of Antp-QBP1 (10 µM) used in the INS-1 β-cell assays suggests a potentially higher efficiency compared to other inhibitors. For instance, the FLPNF candidate under development required 200 µM to inhibit hlAPP-induced cytotoxicity in cultured cells (Shi et al., 2019, A novel pentapeptide inhibitor reduces amyloid deposit formation by direct interaction with hIAPP. Int. J. Endocrinol. 2019). Another potent inhibitor, D-ANFLVH, required a 10-fold higher molar ratio than full-length IAPP to completely abolish IAPP-mediated toxicity in Rin1056A β-cells (Potter et al., 2009, Amyloid inhibitors enhance survival of cultured human islets. Biochim. Biophys. Acta - Gen. Subj. 1790, 566-574). Interestingly, Antp-QBP1 treatment inhibited polyQ inclusion body formation and cytotoxicity in COS-7 cells at a final concentration of 20 µM, which is twice the concentration used in our INS-1 β-cell assays.

## Claims

1. A peptide comprises an amino acid sequence with at least a 70% of identity with SEQ ID NO: 1 or NO:2, for use in the treatment and/or prevention of diabetes.

2. The peptide for the use according to claim 1, wherein the peptide comprises, preferably consists of, the amino acid sequence SEQ ID NO: 1 or SEQ ID NO:2.

3. The peptide for use according to claim 1 or 2, wherein the peptide is comprised in a composition.

4. The peptide for use according to claim 3, wherein the composition further comprises an excipient and/or an acceptable pharmaceutical carrier and/or an adjuvant, or any combinations thereof.

5. The peptide for use according to claim 3 or 4, wherein the composition it is presented in an adapted form for oral, parenteral, intramuscular, intraarterial, intravenous, subcutaneous, epidural, intradermic intraperitoneal or intravesicular administration.

6. The peptide for use according to any one of claims 1 to 5, wherein diabetes is type 2 diabetes mellitus.
